# EUROPEAN PATENT SPECIFICATION

(11) **EP 1 080 211 B1**
(45) Date of publication and mention of the grant of the patent: **14.07.2010**
(21) Application number: 99927745.2
(22) Date of filing: 21.05.1999
(51) Int. Cl.: C12N 15/74, C12N 15/54, C12N 15/31, C12N 9/10, C07K 14/245, C07K 14/28, C07K 14/435, A61K 39/106, A61K 39/108

(54) **METHOD OF PRODUCING THY-A-DEFICIENT STRAINS OF VIBRIO CHOLERAE, SUCH STRAINS AND THEIR USE**
VERFAHREN ZUR HERSTELLUNG THY-A DEFIZIENTE VIBRIO CHOLERAE STÄMME, SOLCHE STÄMME UND DEREN VERWENDUNG
METHODE DE PRODUCTION DE SOUCHES $i(THY) A?- DE $i(VIBRIO CHOLERAE), LESDITES SOUCHES ET LEUR UTILISATION

(30) Priority: 26.05.1998 SE 9801852
(43) Date of publication of application: 07.03.2001
(73) Proprietor: Crucell Sweden AB, 171 63 Solna (SE)
(72) Inventor: CARLIN, Nils, S-165 57 Hässelby (SE); LEBENS, Michael, R., S-413 22 Göteborg (SE)
(74) Representative: Verhage, Richard Abraham
(86) International application number: PCT/EP1999/003509
(87) International publication number: WO 1999/061634

(56) References cited:
- EP-A- 0 251 579
- EP-A- 0 406 003
- WO-A-94/19482
- WO-A-99/35271
- US-A- 5 470 729
- DATABASE EMPRO2 [Online] E.M.B.L. Databases Accession Number: Y17135, 1 May 1998 (1998-05-01) VALLE E ET AL: "Vibrio cholerae thyA gene" XP002118053
- ATTRIDGE SR: "Thymine auxotrophy as an attenuating marker in Vibrio cholerae" MICROB PATHOG, vol. 19, no. 1, July 1995 (1995-07), pages 11-18, XP002118052
- DATABASE EMPRO2 [Online] E.M.B.L. Databases Accession Number: AJ006514, 5 June 1998 (1998-06-05) CARLIN N: "Vibrio cholerae lgt and thyA genes" XP002118055
- DATABASE EMPRO2 [Online] E.M.B.L. Databases Accession Number: AJ010968, 21 September 1998 (1998-09-21) CARLIN N ET AL: "Vibrio cholerae nptA gene" XP002118054
- DATABASE EMBL [Online] EBI 05 June 1998 CARLIN N I A: 'Vibrio cholerae lgt and thyA genes' Retrieved from EMBL Database accession no. AJ006514

## Description

The present invention relates to a method of producing *thy* A' strains of *Vibrio cholerae*, such strains and their use. The invention particularly relates to a strain of *Vibrio cholerae* that has been deprived of its *thy* A gene in the chromosome, i.e. a Δ *thy* A strain lacking the functionality of the *thy* A gene. This strain may comprise one or several episomal autonomously replicating DNA elements, such as plasmids, having an optionally foreign, e.g. *E. coli,* functional *thy* A gene that enables the strain to grow in the absence of thymine in the growth medium, and optionally having a structural gene encoding a homologous or heterologous protein. Also disclosed are *thy* A nucleotide sequences and proteins encoded by them, and a vaccine comprising as an immunizing component a *Vibrio cholerae* Δ *thy* A strain of the invention or a *thy* A strain of *V. cholerae* produced by the method disclosed herein.

### Background.

The expression of recombinant genes in bacterial hosts is most often achieved by the introduction of episomal self-replicating elements (*e*.*g*. plasmids) that encode the structural gene of the protein of interest under the control of an appropriate promoter, into host bacteria. Such plasmids are most commonly maintained by the inclusion of selective marker genes that encode proteins that confer resistance to specific antibiotics (such as ampicillin, chloramphenicol, kanamycin, tetracycline etc.). They are then maintained in the host by addition of the appropriate antibiotic to the culture medium.

Stable maintenance of plasmids in host strains often requires the addition of the appropriate antibiotic selection without which they may segregate out giving rise to significant numbers of cells in any culture, that are devoid of plasmid and therefore cannot express the desired product.

However, the use of antibiotics in the production of recombinant proteins is undesirable for a number of reasons. Apart from the obvious increase in costs arising from the need to add them as a supplement to the growth medium, the use of antibiotics is considered a problem in the production of any recombinant protein intended for human or veterinary use. This is primarily for three reasons. Firstly, residual antibiotics can, in sensitive individuals, cause severe allergic reactions. Secondly, there is the possibility of selection for antibiotic resistant bacteria in the natural bacterial flora of those using the product, and finally, DNA encoding the antibiotic resistance may also be transferred to sensitive bacteria in individuals using the product, thereby also spreading undesired antibiotic resistance in a cohort.

There are already inventions dealing with this problem, one such is the *par* gene which will effectively kill all cells that do not retain a copy of the plasmid after each cell division [1].

Another patent application [2], which touches on the invention described herein, was based on the knowledge of the *thy*A DNA sequence in *E. coli*. The authors introduced the *thy*A gene on a plasmid but used host strains that were spontaneous *thy*A⁻ mutants selected on the bases of trimethoprim resistance. Such mutants are not vell defined (carrying point mutations or small deletions) and may revert to the wild-type (*i*.*e*. *thy*A⁺) at unacceptably high frequencies. This would lead to that the host bacteria could eliminate the plasmid and hence lose, or not give consistent and reliable, production of the desired recombinant product. An additional problem with trimethoprim selection is the possibility that resulting thymine dependence may arise due to a mutation in the dihydrofolate reductase (*fol*A) gene and hence not be complemented by a plasmid-bome *thy*A gene [3]. This patent application has been discontinued at least in Europe.

The use of *V.cholerae* for expression of recombinant genes has been shown to be advantageous over other prokaryotic expression systems in common use in that specific recombinant products may be produced in large quantities and secreted into the culture medium, thereby facilitating downstream purification procedures. This is in contrast to *E.coli* where the product often assembles in the periplasmic space [4]. One important factor endowing *V.cholerae* with this property is the *eps* genes in *V.cholerae* [5].

Thymidylate synthetase encoded by the *thy*A gene of *Escherichia coli* and other bacteria catalyses the methylation of deoxyuridylate (dUMP) to deoxythymidylate (dTMP) and is an essential enzyme in the biosynthesis of deoxyribothymidine triphosphate (dTTP) for incorporation into DNA. In the absence of this enzyme the bacteria become dependent upon an external source of thymine which is incorporated into dTTP by a salvage pathway encoded by the *deo* genes [6].

Spontaneous mutants that arc *thy*A⁻ can be readily isolated on the basis of trimethoprim resistance. This antibiotic inhibits tetrahydrofolate regeneration from dihydrofolate produced by thymidylate synthetase-calalysed dTMP synthesis. Thus, if the cells are *thy*A⁻ they become thymine dependent but no longer deplete the tetrahydrofolate pool in the presence of trimethoprim.

### Description of the invention

The present invention is, in its different aspects, based on the novel nucleotide sequence of the *thy*A gene in *Vibrio cholerae*. A useful application of the *thy*A gene is e.g. in maintenance of recombinant plasmids employed in the overproduction of recombinant proteins in *V. cholerae*, and in the use of the sequence for insertion of foreign genes in a selectable and site-specific manner into the *V. cholerae* chromosome.

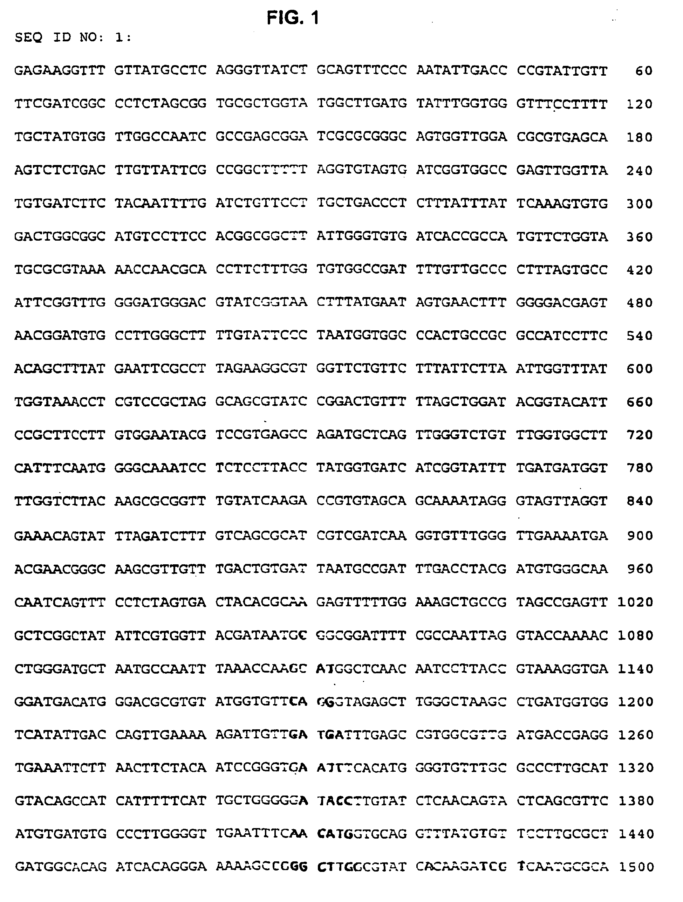

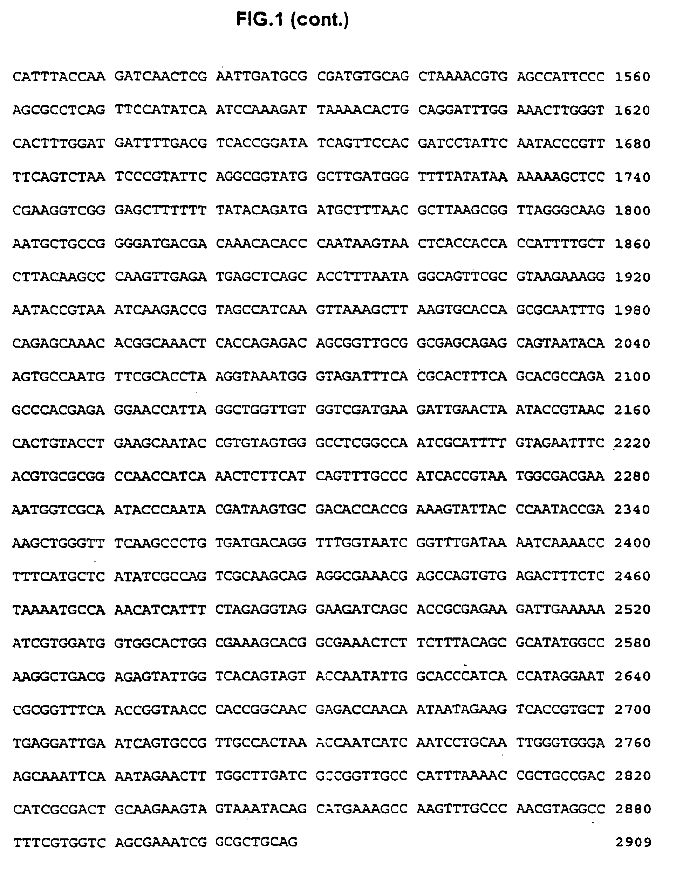

A method of producing a *thy* A⁻ strain of *Vibrio cholerae* comprises the step of site-directed mutagenesis in the *V. cholerae* chromosome for the deletion and/or insertion of gene nucleotides at the locus of the *thy* A gene having essentially the nucleotide sequence SEQ ID NO: 1 of FIG 1.

The expression "having essentially the nucleotide sequences" in this specification is intended to comprise nucleotide sequences which have some natural or unnatural nucleotide extensions, truncations, deletions or additions that do not interfere with the natural function of the nucleotide sequence in question.

*A Vibrio cholerae thy* A⁻ strain which is a Δ *thy* A strain lacking the functionality of the *thy* A genes is described herein. The Δ *thy* A⁻ strain of *V. cholerae* may comprise one or several episomal automously replicating DNA elements having a functional *thy* A gene that enables the strain to grow in the absence of thymine in the growth medium. The episomal autonomously replicating DNA element may be a plasmid. The Δ *thy* A strain may comprise in an episomal autonomously replicating DNA element, especially a plasmid, a foreign *thy* A gene, such as an *E. coli* gene.

The Δ *thy* A strain described herein may comprise in one or several episomal autonomously replicating DNA elements, especially plasmids, in addition to a foreign *thy* A gene, such as an *E. coli* gene, also a structural gene encoding a homologous or heterologous protein, such as heat labile enterotoxin B-subunit of *Escherichia coli* (LTB) or *Schistosoma japonicum* glutathione S-transferase 26 kD protein (GST26kD).

According to the invention there is thus provided an expression system for use in the preparation of a homologous or heterologous protein, which expression system comprises a stable *Vibrio cholerae* strain harbouring an expression vector,
wherein the said stable *Vibrio cholerae* strain lacks the functionality of a *thy*A gene and the said expression vector comprises a functional *thy*A gene and at least one gene encoding a homologous or heterologous protein.

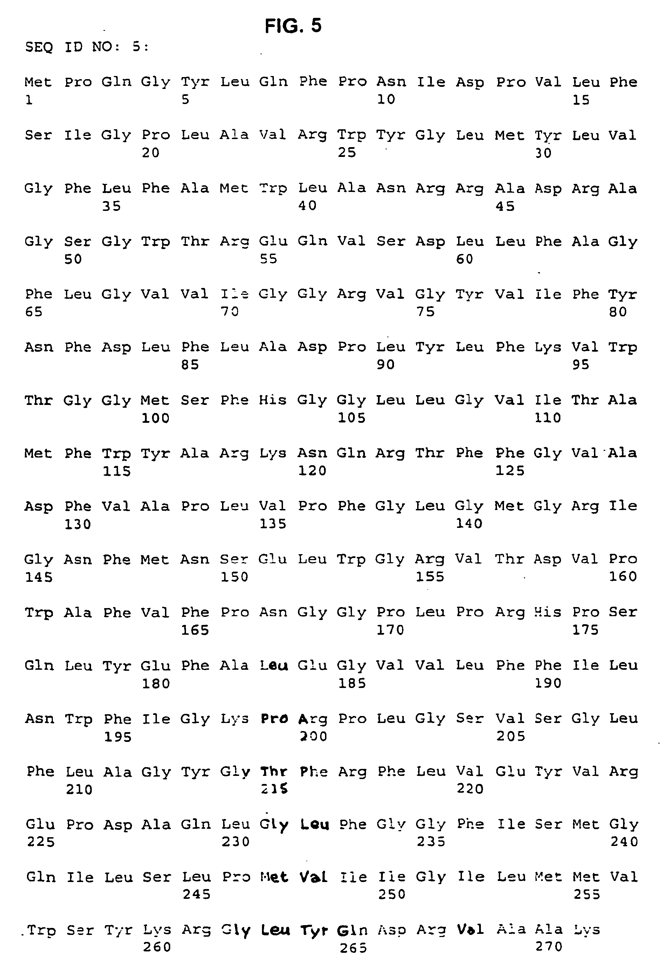

Also disclosed are:
- a process for the preparation of a homologous or heterologous protein, which process comprises:
   maintaining an expression system as disclosed herein under conditions which permit the expression of the homologous or heterologous protein; and
   recovering the expressed heterologous or homologous protein.
- a homologous or heterologous protein obtainable by such a process which is free from antibiotic residues;
- a method for the preparation of a stable *Vibrio cholerae* strain which lacks the functionality of a *thy*A gene, which method comprises:
   transfecting or transforming a *Vibrio cholerae* cell with a vector as described below; and
   selecting a cell which lacks the functionality of a *thy*A gene.
- a stable *Vibrio cholerae* strain obtainable by such a method; and
- a method for the preparation of an expression system for use in the preparation of a homologous or heterologous protein, which method comprises:
   preparing a stable *Vibrio cholerae* strain which lacks the functionality of a *thy*A gene using the method as set out above; and
   transfecting or transforming the stable *Vibrio cholerae* strain so-prepared with an expression vector which comprises a functional *thy*A gene and at least one gene encoding a homologous or heterologous protein;
- a nucleotide sequence of a 5'- flanking region of a structural *thy* A gene of *Vibrio cholerae* having essentially the nucleotide sequence of SEQ ID NO: 2;
- a nucleotide sequence of a 3' - flanking region of a structural *thy* A gene of *Vibrio cholerae* having essentially the nucleotide sequence of SEQ ID NO; 3;
- an isolated nucleic acid sequence of from about nucleotide 14 to about nucleotide 838 of SEQ ID NO: 1.
- an isolated nucleic acid sequence comprising the sequences of from about nucleotide 1 to about nucleotide 838 of SEQ ID NO: 1 and from about nucleotide 1688 to about nucleotide 2909 of SEQ ID NO: 1.
- a vector comprising such a nucleic acid;
- an isolated nucleic acid having the sequence:
   GCTCTAGAGCCTTAGAAGGCGTGGTTC;
   GCTCTAGAGCTACGGTCTTGATTTACGGTAT;
   GGGGGCTCGAGGCGCACATCACATGAA;
   CCCCCCTCGAGCGCCAGAGTTGTTTCTGAA;
   CGGGGTACCTGGCTTGATGGGTTTTAT;
   GAAGGCCTTCGCCTCTGCTTGCGACT;
   GGACTAGTGGGTTTCCTTTTGCTAT; or
   CCCCGCTCGAGACCCTATTTTGCTGCTAC; and
- a protein encoded by a nucleotide sequence of a 5'- flanking region of a structural *thy* A gene of *Vibrio cholerae* as disclosed herein such as the protein having the amino-acid sequence SEQ ID NO: 5 of FIG 5.

Such a protein is useful for research purposes, and a potential target for antimicrobial therapy.

The nucleotide sequence SEQ ID NO: 1 as disclosed herein, is useful for insertion of foreign genes in a selectable and site-specific manner into the *V. cholerae* chromosome, and for site-directed mutagenesis in the production of *Vibrio cholerae thy A⁻* strains.

Also disclosed is a vaccine comprising as an immunising component a homologous or heterologous protein according to the invention or a stable *Vibrio cholerae* strain of the invention which lacks the functionality of a *thy* A gene.

Such a vaccine may be used for prophylactic and therapeutic treatment of cholera and optionally other infectious diseases, especially in cases where the strain used has been engineered to express foreign proteins. Such a vaccine will in addition to the immunising component(s) comprise a vehicle, such as physiological saline solution, and other components frequently used in vaccines such as buffers and adjuvants. Useful vehicles, butters, adjuvants and other components are disclosed in e.g. the European and US Pharmacopoeia.

### Short description of the drawings

Figure 1 shows the nucleotide sequence SEQ ID NO: 1 of the *thy* A gene of *Vibrio cholerae.*

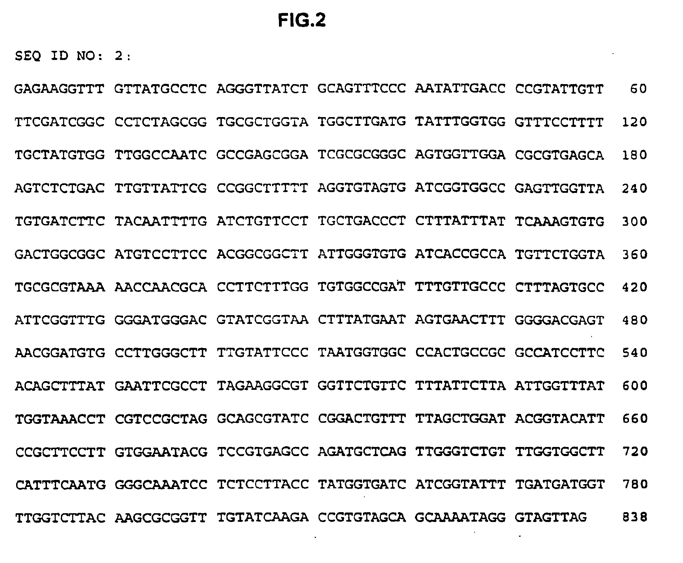

Figure 2 shows the nucleotide sequence SEQ ID NO: 2 of the 5'-flanking region of the structural *thy* A gene of *Vibrio cholerae.*

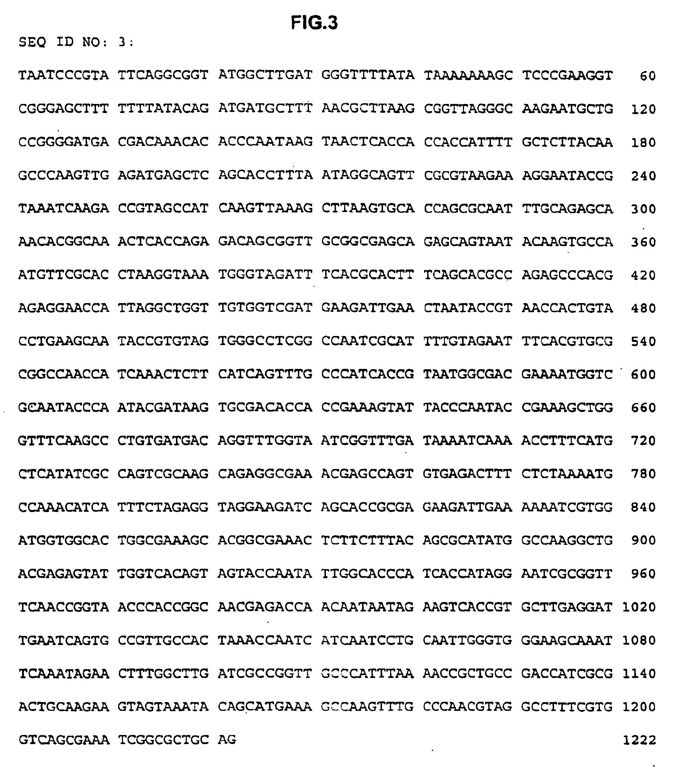

Figure 3 shows the nucleotide sequence SEQ ID NO: 3 of the 3'-flanking region of the structural *thy* A gene of *Vibrio cholerae.*

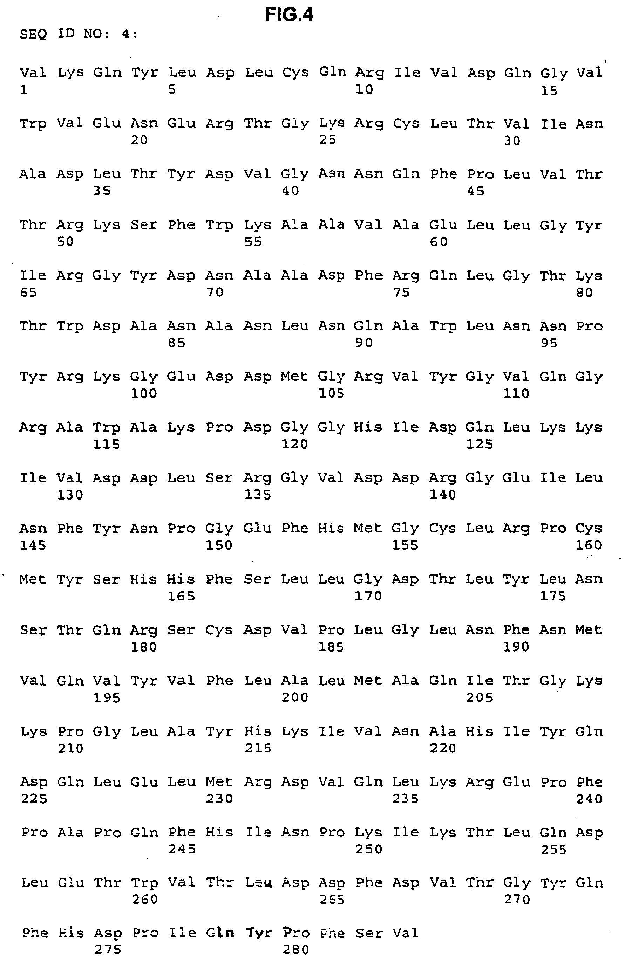

Figure 4 shows the amino-acid sequence SEQ ID NO:4 of the protein encoded by the structural *thy* A gene of *Vibrio cholerae.*
Figure 5 shows the amino-acid sequence SEQ ID NO:5 of the protein encoded by the 5'-flaking region of the structural *thy* A gene of *Vibrio cholerae.*
Figure 6 shows the cloning of a *Eco*RI/*Hind*III fragment containing the *V.cholerae thy*A gene in pUC19.
Figure 7 shows a comparison of *thy*A gene products from *E. coli* [16], *V.*
*cholerae* and *H. influenzae* [17] showing the high degree of homology between *V. cholerae* and *H. influenzae* compared with *E. coli.*
Figure 8 shows the insertion of a Kan^{R}-resistance gene block in the *Pst*I site of the *V. cholerae thy*A gene in pUC 19.

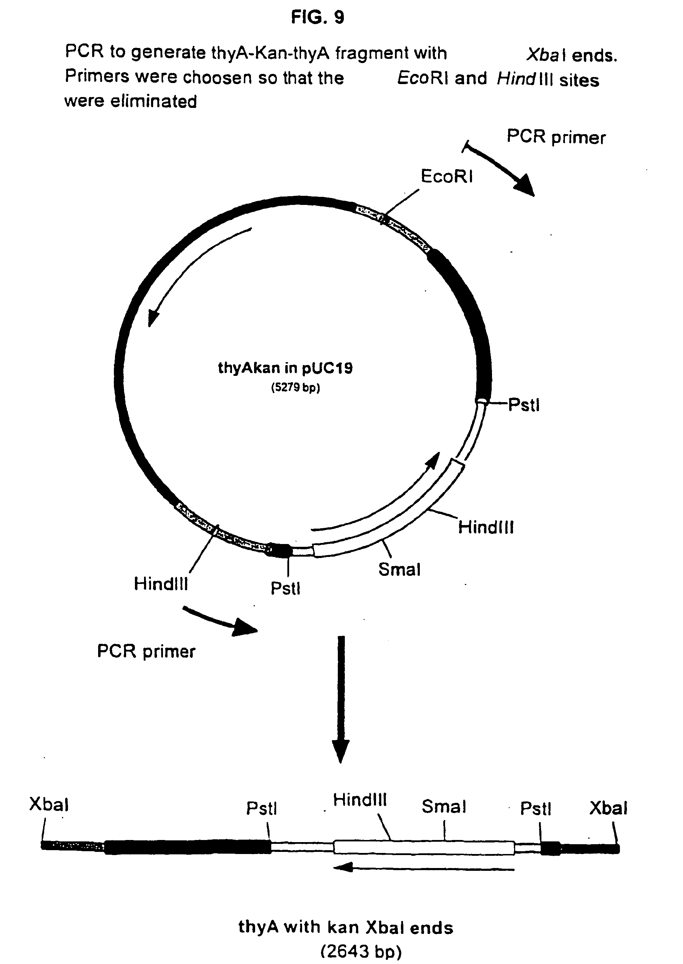

Figure 9 shows PCR to generate a *thy*A -Kan fragment with *Xba*I ends.

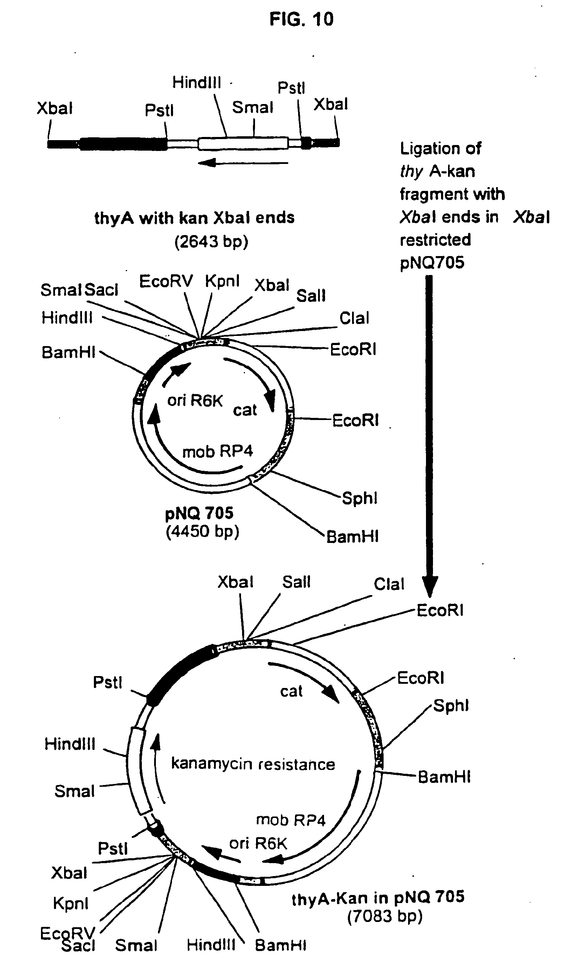

Figure 10 shows ligation of the thyA-Kan fragment with *Xba*I ends in plasmid pNQ705.

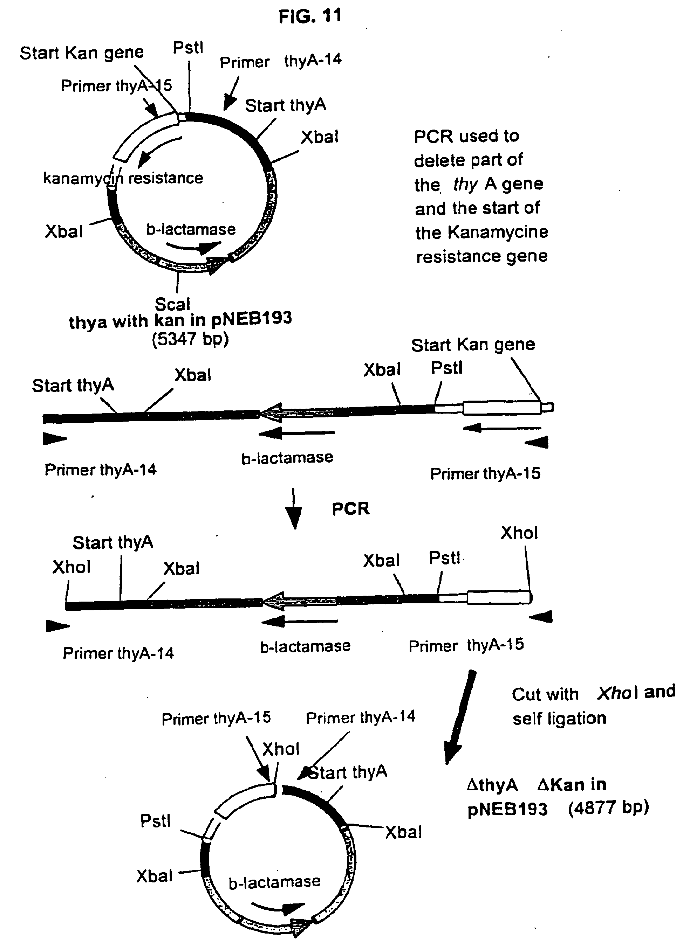

Figure 11 shows partial deletion of the *thy*A gene and the start of the Kan gene in pNEB193.
Figure 12 shows *Xba*I cleavage to excise the Δ*thy*A Δkan gene from pNEB193, ligation into *Xb*aI restricted pDM4.
Figure 13 shows an outline of a strategy to completely delete the *thy*A gene of *V. cholerae.*
Figure 14 shows insertion of the 5' region upstream of *thy*A in pMT-SUICIDE 1; generation of pMT with 5 prim.
Figure 15 shows insertion of the 3' region downstream of *thy*A in pMT with 5 prim; generation of pMT Δ*thy*A *V.cholerae*.
Figure 16 shows the expression vector pMT-eltB(thyA) used for expression of LTB in *V. cholerae* JS 1569 Δ*thy*A*.*
Figure 17 shows the expression vector pMT-GST(thyA) used for expression of GST in *V. cholerae* JS1569 Δ*thy*A.

### Description of experiments

### Strategy employed

In order to produce defined *thy*A mutants of *V. cholerae* that could be used as suitable production strains for recombinant proteins encoded on plasmids maintained by *thy*A complementation, it was first necessary to clone and characterise the wild-type gene and its 5' and 3' flanking regions. Our strategy was to first clone the *thy*A gene of *V. cholerae* on a plasmid, on the basis of complementation of the *thy*A auxotrophy in a strain of *E.coli* K12. Restriction analysis and subcloning experiments were done in order to locate the *thy*A structural gene on the large DNA fragment initially obtained. The appropriate region containing the *thy*A gene and it's 5' and 3' flanking regions gene was then sequenced.

To verify that one of the sequenced genes was in fact the *thy*A gene of *V. cholerae*, homology comparisons were made with *thy*A sequences from other organisms. The cloned gene could also complement the *thy*A phenotype of a *V. cholerae* mutant strain that had been selected on the basis of trimethoprim resistance. Sequence analysis of this mutant showed that it did indeed have a single base change in the gene we had identified as *thy*A, which resulted in a stop codon giving a non-functional truncated gene product.

Knowledge of the *thy*A sequence and that of the region surrounding it allowed the use of suitable suicide vectors for site-directed mutagenesis. Strategies considered were (a) insertional inactivation (b) a combination of insertional inactivation and gene deletion and (c) removal of the entire gene:
(a) Insertional inactivation of the *thy*A gene was achieved by insertion of a Kan^{R} gene block (with the suicide vector pNQ705 [14].
(b) A deletion of approximately 400 bp was made in the strain carrying the Kan^{R} geneblock that removed 200 bp each from the *thy*A gene upstream of the insertion site and from the kanamycin resistance gene which was thereby inactivated. We thus obtained a deleted *thy*A gene where the deletion was in the central part of the gene and followed by an insertion of a non-coding region of DNA. This construct was inserted into the *V. cholerae* chromosome using the suicide vector pDM4 and resulted in a strain called JS1569 Δ*thy*AΔKan.
(c) Complete removal of the *thy*A gene was done by ligating together the regions flanking the structural gene, taking care not to disrupt other open reading-frames (disruption of the adjacent *lgt* gene is also lethal). The DNA carrying the deletion was cloned into a novel suicide vector (PMT-SUICIDE-1) used for insertion of the sequence into the *V. cholerae* chromosome. The resulting strain is called JS1569 Δ*thy*A.

For expression of recombinant genes in these Δ*thy*A strains of *V. cholerae,* two expression vectors were constructed. Each consisted of the *thy*A gene from *E. coli*, the origin of replication of the general purpose high copy-number vector pUC 19, the *tac* promotor and the rho-independent *trp*A transcription terminator. In one of the two vectors the *lac*l^{q} gene had been inserted in order to regulate expression from the *tac* promotor which also contained the *lac* operator sequence.

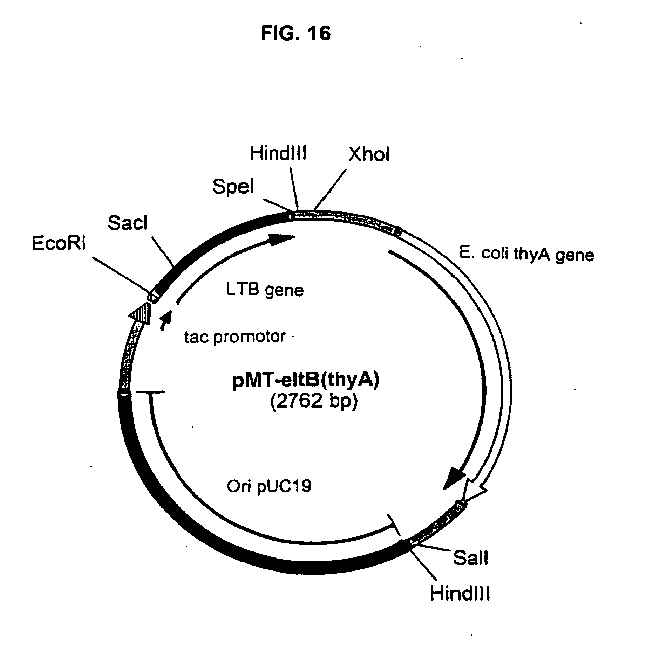

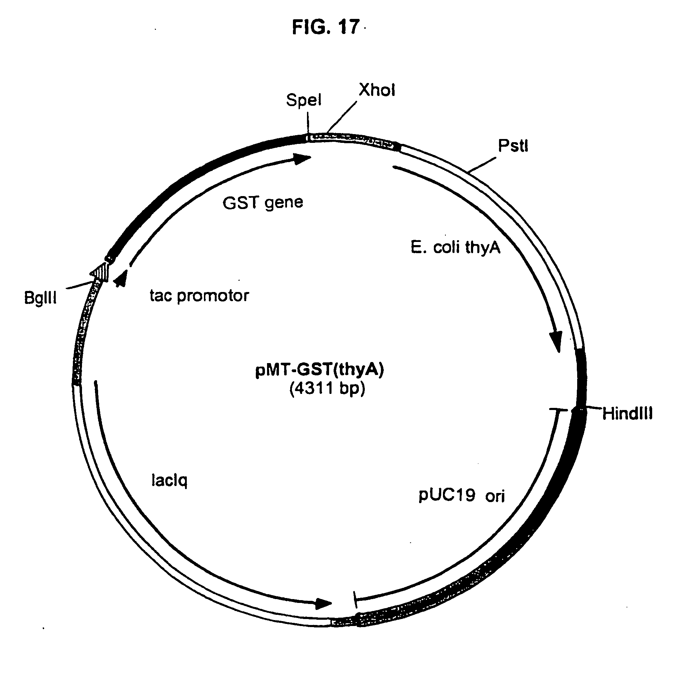

Two genes were cloned into these plasmids and expressed in the newly generated *thy*A -deleted strain of *V. cholerae*; JS1569Δ*thy*A. The first encoded the B subunit of human heat-labile enterotoxin from *E. coli* (LTB) (Figure 16), the second was the sj26 glutathione-S-transferase (GST) from *Schistosoma japonicum* (Figure 17).

LTB is similar in structure to the B subunit of cholera toxin naturally produced by the host strain and was secreted into the growth medium. The other protein is eukaryotic in origin, coming from the Asian Liver Fluke. Sj26 GST is known to express to high levels in *E. coli* and accumulates in the cytoplasm. Expression of the two recombinant proteins was assessed on the basis of GM1 ELISA of the culture supernatant in the case of LTB and a commercially available assay in the case of GST. Both proteins were also analysed on the basis of SDS-PAGE and Western blots.

### Origin of the thyA gene

The *thy*A gene was cloned from strain ***V.cholerae*** JS 1569. This strain originates from the *V.cholerae* Inaba strain 569B of the classical biotype (ATCC No 25870). The strain has a deletion in the *ctx*A gene [7] and has been made rifampicin resistant [8].

### Cloning of a 1.4 kB HindIII/EcoRI fragment encompassing the V.cholerae thyA gene.

Chromosomal DNA prepared by the CTAB method [9] was digested to completion with the restricion enzyme *Hind*III.

The digested DNA was ligated into the general purpose vector plasmid pBR322 (New England Biolabs Inc. Beverly, MA USA) which had been digested with *Hind*III and treated with alkaline phosphatase.

The ligation mixture was electroporated [10] into a *E.coli* HB101 strain that was phenotypically ThyA⁻ (selected on the basis of trimethoprim resistance) and the culture spread onto modifed Syncase (MS) agar plates [11] supplemented with 50 µg/ml ampicillin, but containing no thymine. Thus transformants were selected both on the basis of plasmid acquisition and the presence of a functional *thy*A gene.

Colonies that grew up were streaked out to single colonies on the same type of agar plates, and then grown up in MS broth supplemented with ampicillin. Plasmid DNA was prepared by "Wizard miniprepps" (ProMena Corp. Madison Wis.) and digested with *Hind*III. A fragment of approx. 10-12 kB was isolated, this clone was namned ThyA B2.

To reduce the size of the fragment, the plasmid was cut with *Eco*RI and religated using T4 ligase. The ligated DNA was again electroporated into the *E.coli* strain described above using the same selective conditions for growth of transformants.

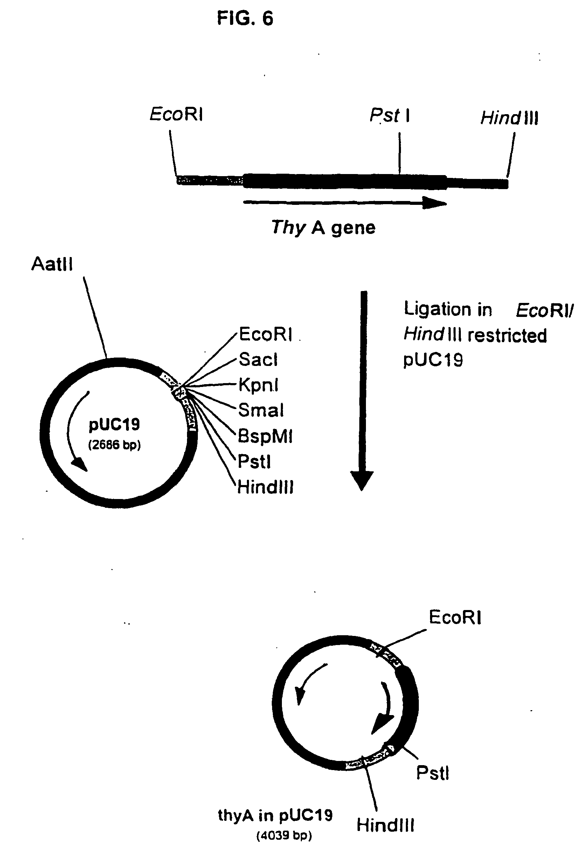

Colonies resulting from this experiment were isolated as described above and plasmid DNA purified and analysed by double digest with *Eco*RI and *Hind*III. A DNA fragment of approximately 1.4 kb remained which retained the ability to complement the *thy*A mutation in the *E. coli* host strain. This fragment was cloned into the plasmid pUC 19 (New England Biolabs) that had been digested with the same two enzymes and treated with alkaline phosphatase. Following electroporation, transformants from the experiment were isolated and characterised as described above. This clone was called ThyA 1:2 (Figure 6).

**Verification that the 1.4 kB *Hind*III/*Eco*RI fragment contains the *thy*A gene.**

Southern blot analysis. To verify that the cloned fragment was indeed from *V.cholerae* chromosomal origin, DNA from strain JS1569 was digested to completion with *Hind*III and *Eco*RI and *Hind*III. The DNA fragments ere resolved by agarose electrophoresis together with *Hind*III digested clone ThyA B2 and *Eco*RI and *Hind*III digested clone ThyA 1:2.

After electrophoresis the DNA was transferred to a Nylonmembrane, immobilised by UV irradiation and hybridised (under stringent conditions) with the 1.5 kB fragment excised from clone ThyA 1:2 that had been labelled with ³²P dCTP using Amershams Multiprime kit.

**Results.** In both *Hind*III digested chromosomal DNA and in *Hind*III digested clone ThyA B2 an approx. 10 kB band was evident. Likewise in *Eco*RI/*Hind*III digested chromosomal DNA and clone ThyA 1:2 plasmid DNA a 1.4 kB band was evident (data not shown). These data demonstrated that the cloned fragment was derived from *V. cholerae* JS1569 DNA.

### Transformation of JS1569 ThyA⁻ with the plasmid ThyA 1:2.

To verify that the 1.4 B cloned *Eco*RI/*Hind*III fragment could support growth of phentotypically ThyA⁻ *V.cholerae,* a thymine dependent mutant of JS1569 (*V.cholerae* JS1569 4.4) was electroporated with the plasmid ThyA 1:2. Electroporation and selective media were as described above. JS1569 4.4 does not grow on MS medium without the addition of thymine.

**Results.** Colonies of JS1569 4.4 were isolated that grew in the absence of thymine. All were shown to harbour the ThyA 1:2 plasmid, thus supporting the assumption that the cloned fragment contained the *thy*A gene from *V.cholerae.*

**DNA sequencing of the plasmid ThyA 1:2.** Plasmid DNA was sequenced by the dideoxy chain termination method [12] using the ABI PRISM™ Dye terminator cycle sequencing kit (Perkin Elmer). Both commercially available as well as custom made primers were used. The DNA sequences were analysed on an ABI PRISM 373 automatic sequencer (Perkin Elmer). Data were analysed using the AutoAssembler Software package (Perkin Elmer). Homology searches with the found DNA sequence were done with the GCG program [13**].**

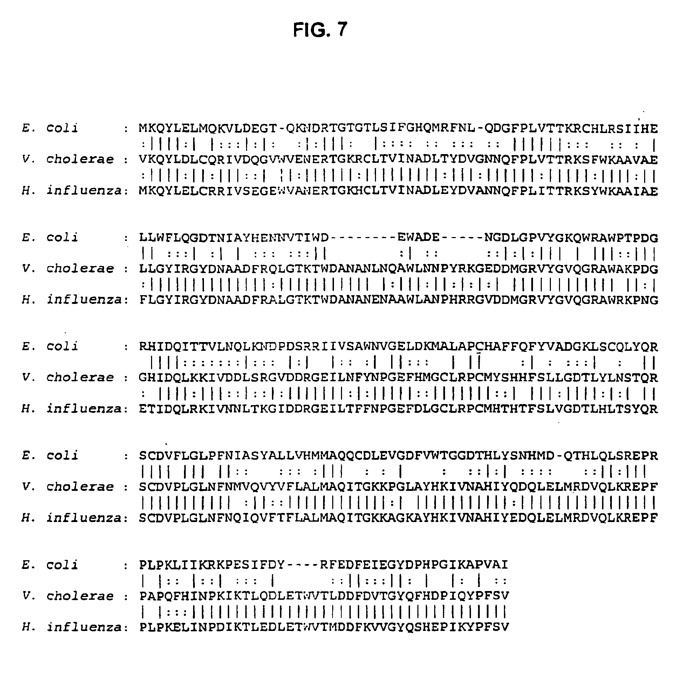

**Results.** The best homologies were with thymidylate synthetases from various species. Note that the homology with *E.coli* thymidylate synthetase is rather weak. (Figure 7)

### Strategy for deletion of the thyA gene in V.cholerae JS1569.

Two different strategies were used for obtaining defined *thy*A mutants of *V. cholerae* JS 1569, the first involved inactivation of the *thy*A gene by insertion of a Kan^{R} gene block followed by partial deletion of the *thy*A gene and the Kan^{R} gene block.

The second strategy was directed to completely delete the *thy*A gene from the chromosome by means of a novel suicide vector pMT SUICIDE-1. This vector contains the 5' and 3' flanking regions of the *thy*A gene as well as the R6K origin of replication and the RP4 *mob* genes.

To replace the *thy*A gene of strain JS1569 we decided to use the already thymine-dependent JS1569 4.4 since preliminary experiments indicated that there is a strong selective disadvantage to go from wildtype to thymine dependence even in the presence of high levels of exogeneous thymine.

### Inactivation of the thyA gene by insertion of a Kan^{R} gene block

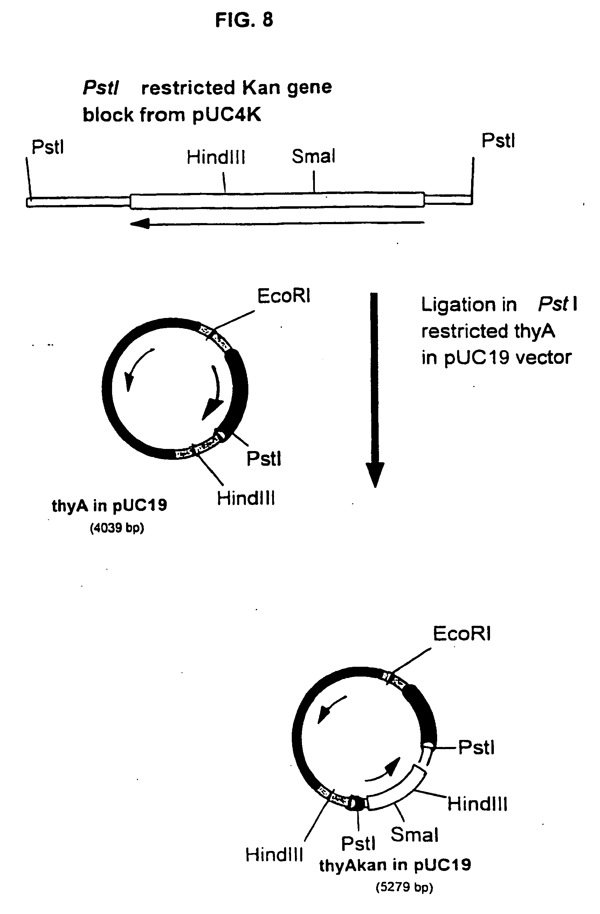

Our strategy involved inactivation of the *thy*A gene by insertion of a kanamycin resistance gene into a unique *Pst*I site in the *thy*A gene in the form of a Kan^{R} gene block (Pharmacia) (Figure 8). This construct was amplified by PCR (Expand™High Fidelity PCR system Boehringer Mannheim) with primers that incorporate *Xba*l ends so that it could be transferred into the suicide plasmid pNQ705 [14] which carries a unique *Xba*I site and the chloramphenicol resistance gene.

The following primers were used for PCR amplification of the insertionally inactiviated gene:

| |
|---|
| ThyA-10: ^{5'}GC**T CTA GA**G CCT TAG AAG GCG TGG TTC^{3'} |
| corresponding to bases 557 to 575 in SEQ m NO: 2 (Figure 2) with an added *Xba*I site |
| (in bold) |
| and |
| ThyA-11: ^{5'}GC**T CTA GA**G CTA CGG TCT TGA TTT ACG GTA T^{3'} |
| corresponding to the complementary sequence of bases 235 to 257 in SEQ ID NO:2 |
| (Figure 3) with an added *Xba*I site (in bold) (Figure 9 + 10). |

The resulting plasmid was then transferred to the *E.coli* S-17 that was used in conjugation experiments.

Since the recipient strain JS1569 4.4 is rifampicin resistant and chloramphenicol sensitive and the donor strain *E.coli* S-17 is both chloramphenicol and kanamycin resistant, transconjugants were selected by selection for resistance to both rifampicin and kanamycin.

The resulting *V. cholerae* strains however would also be chloramphenicol resistant since the entire plasmid would initially be inserted into the chromosome.

Exconjugants that had incorporated the inactivated *thy*A gene carrying the Kan^{R} geneblock into the chromosome and lost the pNQ705 plasmid could then be selected among those that were chloramphenicol sensitive but remained kanamycin resistant.

To verify insertion of the Kanamycin resistance gene in the *thy*A gene the entire *thy*A gene was PCR amplified with primers thyA-10 and thyA-11, and the size of the resulting fragment compared to that of the native *thy*A gene. The expected *thy*A fragment of 2.6 kb compared to that of the native *thy*A gene of 1.4 kb was found.

Results. Exconjugants were shown to be kanamycin resistant, chloramphenicol sensitive and when amplified by PCR, shown to have incorporated the kanamycin resistance gene block into the chromosome. Sequencing of the amplified fragment showed that the only defect in the gene was due to the insertion of the kanamycin gene. This indicated that the recombination event that had incorporated the insertionally inactivated gene into the chromosome had also eliminated the point mutation that had made the recipient strain (JS1569 4.4) thymine dependent. Growth of the resulting strain was only observed if the growth medium was supplemented with thymine (200 µg/ml).

### Partial deletion of the thyA gene and the Kan^{R} gene block

To further ensure a nonreversible *thy*A mutation the insertionally inactivated *thy*A was subcloned as a *Xba*I fragment into pNEB 193 (New England Biolabs). PCR primers were designed that deleted 209 basepairs from the *thy*A gene and removed 261 basepairs from the Kan^{R} geneblock.

Thus the *thy*A gene was further disrupted and that the kanamycin resistance gene was also inactivated (by removal of the start of the coding region). The overall result of this procedure was a strain carrying a deleted *thy*A gene that also contained an insertion of noncoding DNA.

| |
|---|
| ThyA-14: ^{5'}GGG GG**C TCG AG**G GGC ACA TCA CAT GAA^{3'} |
| ThyA-15: ^{5'}CCC CC**C TCG AG**C GCC AGA GTT GTT TCT GAA^{3'} |
| Letters in **bold** indicate *Xho*I cleavage sites (Figure 11). |

After PCR amplification a DNA fragment was obtained encompassing the entire plasmid with exception of the deleted region. The amplified DNA was digested with *Xho*I, self ligated and transformed into *E.coli* HB101. Colonies were selected for on plates containing ampicillin. Individual colonies were selected and restreaked. Small-scale plasmid preparations from individual colonies yielded the expected restriction patterns when anlysed with *Xba*I, *Xho*I, *Hind*III and *Rsa*I restriction enzymes.

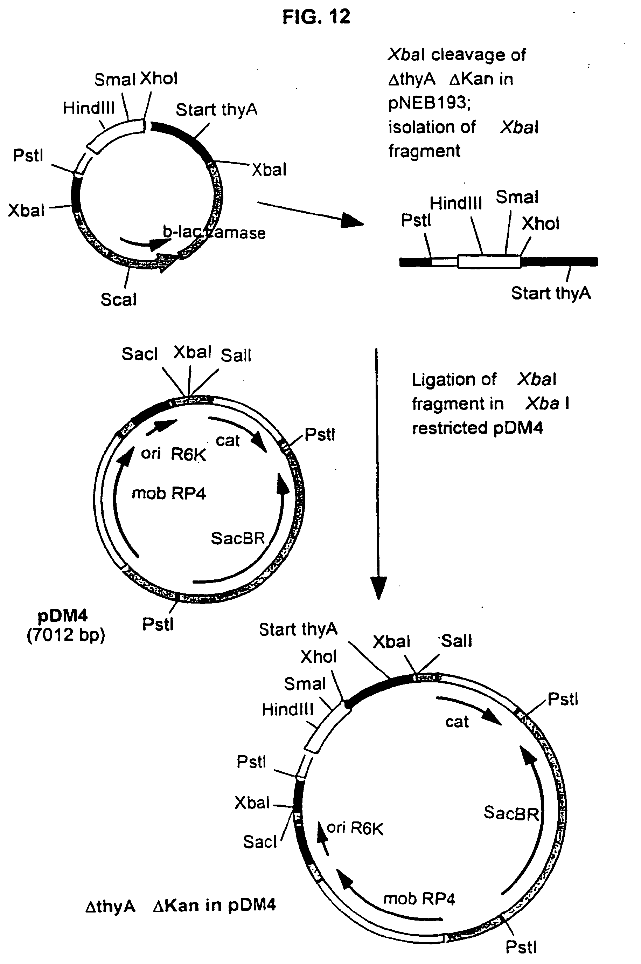

The incomplete *thy*A gene carrying an inactivated kanamycin resistance gene was cut out from the vector by *Xba*I digestion, purified and ligated into pDM4 [15] (Figure 12). PDM4 is a suicide vector derived from pNQ705 containing the *Sac*BR gene from *Bacillus subtilis* and a modified multicloning site.

After transfer of the pDM4 (Δ*thy*AΔKan) plasmid to the *E.coli* S-17 strain a transconjugation experiment was performed. This time the *V.cholerae* JS 1569 *thy*AKan strain obtained above was used as recipient strain.

The mating was done as described above with selection for rifampicin and chloramphenicol. After growth in this medium colonies were selected on medium containing 10% sucrose in the absence of chloramphenicol. Sucrose induces the sacBR gene which encodes levansucrase that converts sucrose to levan. This compound is toxic to many Gram negative organisms. In this way clones still carrying the suicide plasmid were killed leaving exconjugants that had lost the plasmid.

**Results.** A colony was selected that was chloramphenicol and kanamycin sensitive. PCR amplification of the *thy*A region with the primers ThyA-10 and thyA-11 confirmed that the *thy*AKan fragment (2.6 kb) on the chromosome had been replaced with the Δ*thy*AΔKan fragment (2.1 kb).

Growth of the resulting strain was only observed if the growth medium was supplemented with thymine (200 µg/ml). This strain was named *V.cholerae* JS 1569 Δ*thy*AΔKan.

### Direct deletion of the thyA gene in V. cholerae.

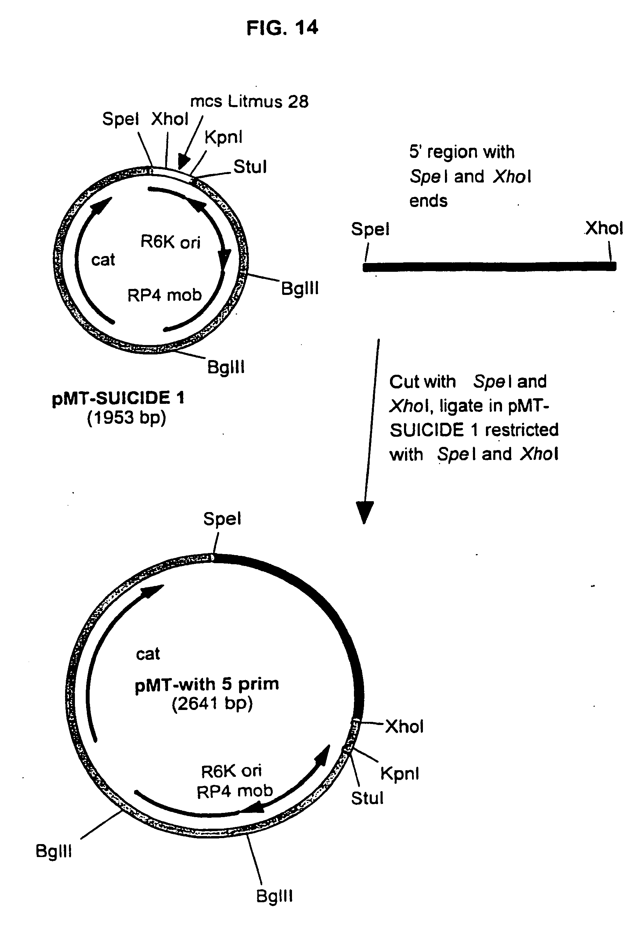

For this approach the 5' and 3' sequences flanking the *thy*A gene were used. A novel suicide vector was constructed, pMT SUICIDE-1 (Fig 14) that contains the R6K origin of replication, the *mob* genes from RP4, a chloramphenicol resistance gene and a multicloning site from Litmus 28 (New England Biolabs). Effectively, a modified fragment was constructed in which the *thy*A coding region was replaced by a multicloning site (derived from Litmus 28) leaving only the 5' and 3' region of the *thy*A locus from *V.cholerae*. The resulting plasmid was used to generate a *V. cholerae* strain in which the entire *thy*A gene had been deleted.

As starting material for this construction the pMT SUICIDE-1 plasmid was used (M. Lebens, unpublished).

From the 5' and 3' regions of the *thy*A locus the following PCR primers were designed:

| |
|---|
| Thy-33: ^{5'}GG**A CTA GT**G GGT TTC CTT TTT GCT AT^{3'} |
| corresponding to bases 109 to 126 in the SEQ ID NO:2 (figure 2) (5' region of the *thy*A region) with a *Spe*I site (indicated in bold) and |
| ThyA-34: ^{5'}CCC CG**C TCG AG**A CCC TAT TTT GCT GCT AC^{3'} |
| corresponding to the complementary sequence of base 815 to 832 in the SEQ ID NO:2 with a *Xho*I site (indicated in bold) attached to it. |
| This primer pair gives a PCR fragment of 743 bases corresponding to the 5' flanking region of the *thy*A gene. |
| ThyA-31: ^{5'}CGG **GGT ACC** TGG CTT GAT GGG TTT TAT^{3'} |
| corresponding to bases 22 to 39 in the SEQ ID NO:3 (figure 3) (3' region of the *thy*A region) with a *Kpn*I site (indicated in bold) and |
| ThyA-32: ^{5'}GA**A GGC CT**T CGC CTC TGC TTG CGA CT^{3'} |
| corresponding to the complementary sequence of bases 731 to 749 in the SEQ ID NO:3 with a *Stu*I site (indicated in bold). |
| This primer pair gives a PCR fragment of 746 bases corresponding to the 3' flanking region of the *thy*A gene. |

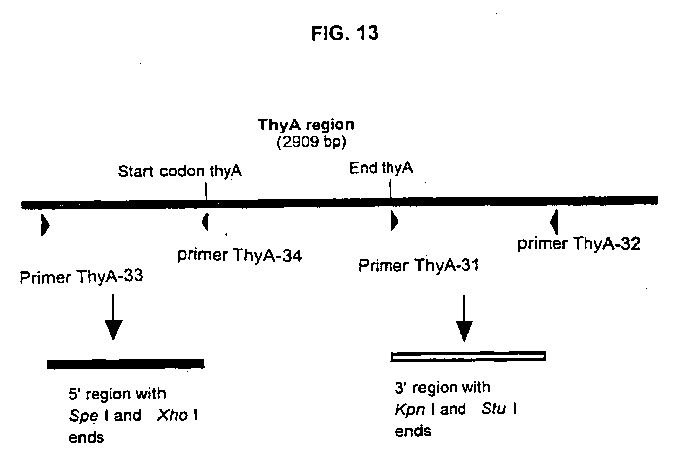

As template for the PCR reactions a chromosomal DNA preparation from *V. cholerae* JS1569 was used (Figure 13).

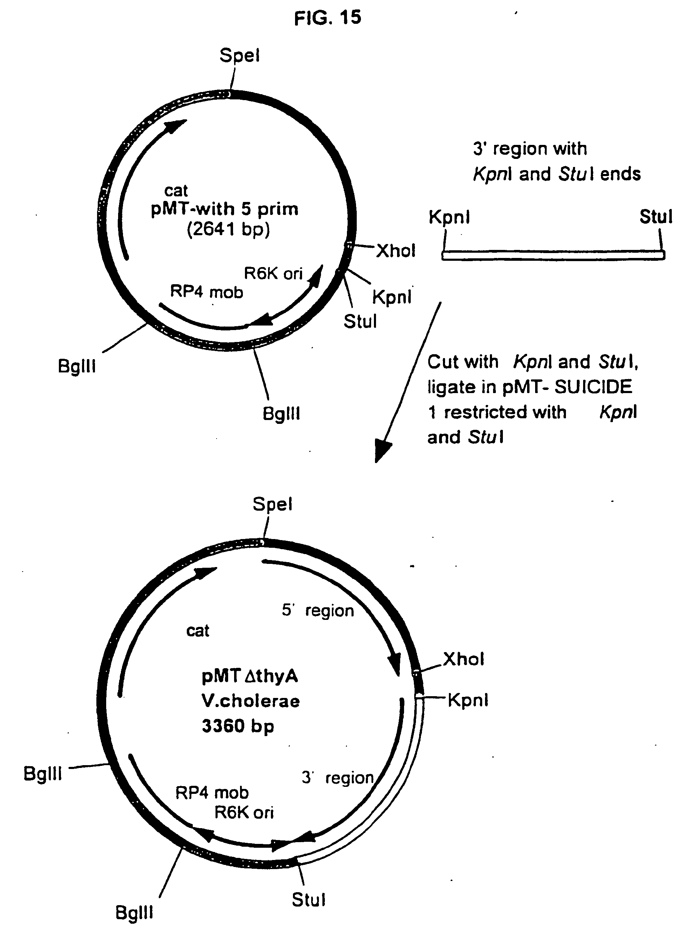

The amplified DNA were digested with the appropriate restriction enzymes and cloned into the pMT-SUICIDE 1 vector (Figure 14 and 15) yielding the plasmid pMTΔ*thy*A *V.cholerae* that contains approximately 700 base-pairs of the 5' region upstreams of the *thy*A gene and the same number of base-pairs of the 3' region downstreams of the *thy*A gene.

This plasmid was transferred to *E.coli* S17-1 and used in conjugation experiments as described above. As recipient the *V. cholerae* JS1569 4.4 strain was used. Matings were done on LB agar supplemented with rifampicin, chloramphenicol and thymine. Exconjugants that had lost the suicide plasmid from the chromosome were selected on the basis of chloramphenicol sensitivity.

**Results**. A chloramphenicol sensitive and rifampicin resistant colony was selected. PCR amplification with the primers ThyA-10 and ThyA-11 of the *thy*A region resulted in a 1.4 kb fragment from the native *thy*A gene and a 0.6 kb fragment from the Δ*thy*A gene.This confirmed that the *thy*A structural gene on the chromosome had been deleted. Furthermore the bacteria could only grow in medium complemented with thymine. This strain is namned *V. cholerae* JS1569 Δ*thy*A.

### Expression of the B subunit of heat-labile enterotoxin from E. coli (LTB) and the sj26 glutathione-S-transferase (GST) from Schistosoma japonicum in V. cholerae JS1569 ΔthyA..

Two expression vectors were constructed, each consisted of the *thy*A gene from *E. coli*, the origin of replication of the high copy-number vector pUC19, the *tac* promotor and the rho-independent *trp*A transcription terminator. In one of the two vectors the *lac*I^{q} gene had been inserted in order to regulate expression from the *tac* promotor which also contained the *lac* operator sequence (figure 16 and 17).

### Expression of the LTB protein in V.cholerae JS1569 ΔthyA strain.

The expression vector shown in figure 16 was electroporated into *V.cholerae* JS 1569 Δ*thy*A. Transformants were selected for on MS -agar. Individual colonies were grown up to produce mini-plasmid preps that were checked by restriction enzyme analysis. For expression a transformant was grown in MS medium at 37°C in a shaker culture. The culture medium was harvested and assayed for LTB by the GM1- ELISA.

**Results.** The culture was found to produce approximately 300 µg/ml of LTB as assayed by the GM1 ELISA. SDS-PAGE and Western blot using an LTB specific monoclonal antibody further verified that the secreted protein was LTB.

### Expression of the GST protein in V.cholerae JS1569 ΔthyA strain

The sj26 glutathione-S-transferase (GST) from *Schistosoma japonicum* was cloned in the expression vector shown in figure 17. This vector is identical to the first except for the sequence of the *lac*I^{q} gene. The *lac*I^{q} allows for controlled expression of recombinant proteins. The vector was electroporated into *V.cholerae* JS 1569 Δ*thy*A. Transformants were selected for on MS -agar. Indivudual colonies were grown up to produce mini-plasmid preps that were checked by restriction enzyme analysis. For expression a transformant was grown in MS medium at 37°C in a shaker culture with addition of IPTG.

**Results.** The recombinant protein was found in the cytoplasm of the *V*. *cholerae* bacteria. SDS-PAGE and Western blot with a GST specific monoclonal antibody (Pharmacia BioTech, Uppsala) confirmed that GST was expressed. The level of GST expression was more difficult to determine than for LTB since the protein was expressed intracellulary but was judged to be in the same range as for LTB.

### References

**1.** Molin, S., K. A. Gerdes. 1984. Stabilized plasmids. US Patent 4,760,022.
**2.** Morona, R., and S. R. Attridge. 1987. Non-antibiotic marker system. EPC-A- 0251579.
**3.** Green, J. M., B. P. Nichols, and R. G. Matthews. 1996. Folate biosynthesis, reduction and polyglutamylation. In: F. C. Neidhardt, R. Curtiss III, J. L. Ingraham, E. C. C. Lin, K. B. Low, B. Magasanik, W. S. Reznikoff, M. Riley, M. Schaechter and H. E. Umbarger (Eds.) Escherichia coli and Salmonella cellular and molecular biology. ASM Press Washington D.C. pp665-673.
**4.** Neill, R. J., B. E. Ivins, and R. K. Holmes. 1983. Synthesis and secretion of the plasmid-coded heat-labile enterotoxin of Escherichia coli in Vibrio cholerae. Science. 221: 289-290.
**5.** Sandkvist, M., M. Bagdasarian, S. P. Howard, and V. J. DiRita. 1995. Interaction between the autokinase EpsE and EpsL in the cytoplasmic membrane is required for extracellular secretion in Vibrio cholerae. EMBO J. 14:1664-1673.
**6.** Neuhard, J. and R. A. Kelln. 1996. Biosynthesis and conversions of pyrimidines. In: F. C. Neidhardt, R. Curtiss III, J. L. Ingraham, E. C. C. Lin, K. B. Low, B. Magasanik, W. S. Reznikoff, M. Riley, M. Schaechter and H. E. Umbarger (Eds.) Escherichia coli and Salmonella cellular and molecular biology. ASM Press Washington D.C. pp580-599.
**7.** Kaper, J. B., H. Lockman, M. M. Baldini, and M. M. Levine. 1984. A recombinant live oral cholera vaccine. Biotechnology 2:345-349.
**8.** Sanchez, J., and J. Holmgren. 1989. Recombinant system for overexpression of cholera toxin B subunit in Vibrio cholerae as a basis for vaccine development. Proc. Natl. Acad. Sci. USA. 86:481-485.
**9.** Wilson, K. 1994. Preparation of genomic DNA from Bacteria. In Current protocols in Molecular Biology (F.A. Ausubel, R. Brent, R.E. Kingston, D.D. Moore, J. G. Seidman, J.A. Smith, and K. Struhl, eds.) pp. 2.4.1-2.4.2 John Wiley & Sons, New York.
**10.** Sheen, J. 1994. High-efficency transformation by electroporation. In Current protocols in Molecular Biology (F.A. Ausubel, R. Brent, R.E. Kingston, D.D. Moore, J. G. Seidman, J.A. Smith, and K. Struhl, eds.) pp. 1.8.4-1.8.5. John Wiley & Sons, New York.
**11.** Lebens, M., S. Johansson., J. Osek., M. Lindblad and J. Holmgren. 1993. Large-scale production of Vibrio cholerae toxin B subunits for use in oral vaccines. Biotechnology. 11:1574-1578.
**12.** Sanger, F., S. Nicklen, and A. R. Coulson. 1977. DNA sequencing with chain-terminating inhibitors. Proc. Natl. Acad. Sci. USA 74:5463-5467.
**13.** Program Manual for the Wisconsin Package. Version 8. September 1994. Genetics Computer Group, 575 Science Drive, Madison Wisconsin.
**14.** Milton, D. L., A. Nordqvist, and H. Wolf-Watz. 1992. Cloning a metalloprotease gene involved in the virulence mechanism of Vibrio anguillarum J. Bacteriol. 174:7235-7244.
**15.** Milton, D. L., R. O'Toole, P. Högstedt, and H. Wolf-Watz. 1996. Flagellin A is essential for the virulence of Vibrio anguillarum J. Bacteriol. 176:1310-1319.
**16.** Belfort, M., G. Maley, J. Pedersen-Lane and F. Maley. 1983. Primary tructure of the Escherichia coli thyA gene and its thymidylate synthase product. Proc. Natl. Acad. Sci. USA 80: 4914-4918.
**17.** Fleischmann, R.. D., Adams, M. D., White, O., Clayton, R. A., Kirkness, E. F., Kerlavage, A. R., Bult,C.J., Tomb, J.-F., Dougherty, B. A., Merrick, J. M., McKenney, K., Sutton, G., FitzHugh, W., Fileds, C. A., Gocayne, J. D., Scott, J. D., Shirely, R.., Liu, L.-I., Glodek, A., Kelley, J.M. Weidman, J. F., Phillips, C. A., Spriggs, T., Hedblom, E., Cotton, M. D., Utterback, T. R., Hanna, M. C., Nguyen, D. T., Saudek, D. M., Brandon, R.. C., Fine, L. D., Fritchman, J. L., Fuhrmann, J. L., Geoghagen N.S.M., Gnehm, C. L., McDonald, L. A., Small, K. V., Fraser, C. M., Smith, H. O., and J. C. Venter. 1995. Whole-genome random sequencing and assembly of Haemophilus influenzae RD. Science 269:496-512.

## Claims

1. A Δ *thy*A strain of *Vibrio cholerae* lacking the entire *thyA* gene in the chromosome which has the nucleotide sequence SEQ ID NO:1, or
a Δ *thy*A strain of *Vibrio cholerae* lacking the functionality of its *thyA* gene which has the nucleotide sequence SEQ ID NO:1 located on the chromosome of said strain, obtainable by a method comprising the steps of:
providing a thymine-dependent *Vibrio cholerae* strain;
performing a site-directed insertion of a kanamycin resistance gene in the form of a Kan^{R} geneblock into a PstI site in the *thyA* gene; and
deleting 209 base pairs from said *thyA* gene upstream of the insertion site and 261 base pairs from the Kan^{R} geneblock, thereby obtaining the Δ *thy*A strain.

2. A Δ *thy*A strain of *Vibrio cholerae* according to claim 1, comprising one or several episomal autonomously replicating DNA elements having a functional *thyA* gene that enables the strain to grow in the absence of thymine in the growth medium and the one or several episomal autonomously replicating DNA elements further comprising a structural gene encoding a protein.

3. A Δ *thy*A strain of *Vibrio cholerae* according to claim 2, wherein the episomal autonomously replicating DNA element is a plasmid.

4. A Δ *thy*A strain of *Vibrio cholerae* according to claim 2, wherein the episomal autonomously replicating DNA elements have a foreign *thyA* gene.

5. A Δ *thy*A strain of *Vibrio cholerae* according to claim 4, wherein the foreign *thyA* gene is an *E*. *coli* gene.

6. A Δ *thy*A strain of *Vibrio cholerae* according to claim 2, wherein the encoded protein is selected from heat labile enterotoxin B-subunit of *Escherichia coli* (LTB) and *Schistosoma japonicum* glutathione S-transferase 26 kD protein (GST 26 kD).

## Patentansprüche

1. Δ *thyA-Vibrio cholerae*-Stamm, dem das gesamte thyA-Gen mit der Nucleotidsequenz SEQ ID NO:1 im Chromosom fehlt, oder
Δ *thyA-Vibrio cholerae*-Stamm, dem die Funktionsfähigkeit des thyA-Gens mit der SEQ ID NO:1 1 lokalisiert auf dem Chromosom dieses Stammes fehlt, erhältlich durch ein Verfahren umfassend die Schritte:
Bereitstellen eines Thymin-abhängigen *Vibrio cholerae*-Stamms;
Durchführen einer ortsspezifischen Insertion eines Kanamycin-Resistenz-Gens in Form eines Kan^{R}-Genblocks in eine PstI-Stelle des thyA-Gens; und
Deletion von 209 Basenpaaren vom *thyA*-Gen stromaufwärts der Insertionsstelle und 261 Basenpaaren vom Kan^{R} Genblock, wodurch der Δ *thyA*-Stamm erhalten wird.

2. Δ *thyA-Vibrio cholerae*-Stamm nach Anspruch 1, umfassend eine oder mehrere episomal autonom replizierende DNA-Elemente, die ein funktionelles thyA-Gen haben, das den Stamm befähigt, in Abwesenheit von Thymin im Wachstumsmedium zu wachsen und wobei das eine oder die mehreren episomal autonom replizierenden DNA-Elemente ferner ein ein Protein codierendes Strukturgen umfassen.

3. Δ *thyA-Vibrio cholerae*-Stamm nach Anspruch 2, wobei das episomal autonom replizierende DNA-Element ein Plasmid ist.

4. Δ *thyA-Vibrio cholerae*-Stamm nach Anspruch 2, wobei die episomal autonom replizierenden DNA-Elemente ein fremdes *thyA*-Gen besitzen.

5. Δ *thyA-Vibrio cholerae*-Stamm nach Anspruch 4, wobei das fremde thyA-Gen ein *E*. *coli*-Gen ist.

6. Δ *thyA-Vibrio cholerae*-Stamm nach Anspruch 2, wobei das codierte Protein ausgewählt ist aus hitzelabiler Enterotoxin-B-Untereinheit von *Escherichia coli* (LTB) und *Schistosoma japonicum*-Glutathion-S-Transferase-26 kD Protein (GST 26kD).

## Revendications

1. Souche Δ *thy*A de *Vibrio cholerae* ne contenant pas le gène *thy*A entier dans le chromosome, qui possède la séquence nucléotidique SEQ ID NO: 1, ou
souche Δ *thy*A de *Vibrio cholerae* ne contenant pas la fonctionnalité de son gène *thy*A, qui possède la séquence nucléotidique SEQ ID NO: 1 située sur le chromosome de ladite souche, que l'on peut obtenir via un procédé comprenant les étapes de :
procuration d'une souche de *Vibrio cholerae* dépendant de la thymine ;
mise en oeuvre d'une insertion dirigée d'un gène procurant une résistance à la kanamycine sous la forme d'un bloc de gènes Kan^{R} dans un site Pstl dans le gène *thy*A ; et
suppression de 209 paires de bases dudit gène *thy*A en amont du site d'insertion et de 261 paires de bases du bloc de gènes Kan^{R}, pour ainsi obtenir la souche Δ *thy*A.

2. Souche Δ *thy*A de *Vibrio cholerae* selon la revendication 1, comprenant un ou plusieurs éléments d'ADN épisomiques répliquant de manière autonome, possédant un gène *thy*A fonctionnel qui permet à la souche de croître en l'absence de thymine dans le milieu de croissance, et lesdits un ou plusieurs éléments d'ADN épisomiques répliquant de manière autonome comprenant en outre un gène de structure encodant une protéine.

3. Souche Δ *thy*A de *Vibrio cholerae* selon la revendication 2, dans laquelle l'élément d'ADN épisomique répliquant de manière autonome est un plasmide.

4. Souche Δ *thy*A de *Vibrio cholerae* selon la revendication 2, dans laquelle les éléments d'ADN épisomiques répliquant de manière autonome possèdent un gène *thy*A étranger.

5. Souche Δ *thy*A de *Vibrio cholerae* selon la revendication 4, dans laquelle le gène *thy*A étranger est un gène de *E*. *coli*.

6. Souche Δ *thy*A de *Vibrio cholerae* selon la revendication 2, dans laquelle la protéine encodée est choisie parmi une sous-unité de l'entérotoxine B thermolabile de *Escherichia coli* (LTB) et la protéine glutathion S-transférase de 26 kD de *Schistosoma japonicum* (GST 26 kD).
